# EUROPEAN PATENT SPECIFICATION

(11) **EP 1 691 851 B1**
(45) Date of publication and mention of the grant of the patent: **27.05.2009**
(21) Application number: 04813086.8
(22) Date of filing: 03.12.2004
(51) Int. Cl.: A61L 15/58, A61L 15/42, A61L 15/22, A61L 15/60

(54) **WOUND DRESSINGS AND METHODS**
WUNDVERBÄNDE UND VERFAHREN
PANSEMENTS ET PROCEDES CORRESPONDANTS

(30) Priority: 05.12.2003 US 729114
(43) Date of publication of application: 23.08.2006
(73) Proprietor: 3M Innovative Properties Company, St. Paul MN 55133-3427 (US)
(72) Inventor: BURTON, Scott A., Saint Paul, Minnesota 55133-3427 (US); HYDE, Patrick D., Saint Paul, Minnesota 55133-3427 (US); POPKO, Daniel T., Saint Paul, Minnesota 55133-3427 (US)
(74) Representative: Vossius & Partner
(86) International application number: PCT/US2004/040707
(87) International publication number: WO 2005/056069

(56) References cited:
- WO-A-02/066087
- WO-A-20/04080498
- WO-A-20/04080499

## Description

The wound healing process involves the growth of capillaries, fibroblasts, and epithelium into the wound site for building up new tissue. The newly formed tissue is extremely delicate and supersensitive to external influences. If a wound still in progress of regenerating tissue is covered with a dressing composed of a fibrous material, the fibers may easily intermingle with the newly formed tissues and give rise to inflammatory reactions in the wound tissue, which would result in deterioration of the wound healing process. Furthermore, the wound tissue would also be mechanically damaged in connection with removal and change of dressing. To avoid this, it is desirable that the dressing applied to the wound does not adhere to dried wound exudate, or to any coagulum formed.

Wound dressings intended for use during this particularly sensitive stage of the wound healing process are preferably designed so as not to stick to the wound bed. Also, it is desirable if they are pliable and have a soft wound-contacting surface. In addition, it is desirable if they are capable of absorbing excess amounts of wound exudate and/or to allow for the passage of wound exudate into an absorbent body placed over the dressing.

WO 02/066087 discloses an adhesive composition comprising a polymeric matrix and absorbent particles wherein at least a part of the absorbent particles are microcolloid particles having a substantially rounded or spherical shape.

The present invention is directed to polymer compositions that are useful in wound dressings.

In one aspect of the invention there are provided wound dressings comprising an apertured liquid permeable substrate and an absorbent, nonadherent polymer composition that includes: a hydrophobic organic polymer matrix; a plasticizing agent; and hydrophilic organic microparticles.

For certain embodiments, the hydrophobic polymer matrix includes a styrene-isoprene-styrene copolymer, a styrene-butadiene-styrene copolymer, or mixtures thereof. For certain embodiments, the hydrophobic polymer matrix includes a mixture of two or more polymers.

For certain embodiments, the microparticles when in a substantially nonhydrated form have an average particle size of 10 µm or less. For certain embodiments, the microparticles when in a substantially nonhydrated form have an average particle size of 1 µm or less. For certain other embodiments, the microparticles when in a substantially nonhydrated form have an average particle size of 0.5 µm or less.

For certain embodiments, the apertured liquid permeable substrate includes 1 to 225 apertures per square centimeter. For certain embodiments, the apertured liquid permeable substrate includes apertures having an average opening size of 0.1 millimeter to 0.5 centimeter.

For certain embodiments, the microparticles include an amine-containing organic polymer. For certain embodiments, the amine-containing organic polymer microparticles include a quaternary ammonium salt of an organic polymer. The microparticles include a cationic homopolymer of the methyl chloride quaternary salt of 2-(dimethylamino)ethyl methacrylate.

For certain embodiments, the microparticles include a copolymer of sodium acrylate and acrylic acid.

For certain embodiments, the microparticles are in the form of an inverse emulsion.

For certain embodiments, the microparticles are present in an amount of 1 wt-% to 60 wt-%, based on the total weight of the polymer composition.

For certain embodiments, the polymer composition further includes a bioactive agent, such as an antimicrobial agent. For certain embodiments, the polymer composition further includes an additive selected from the group consisting of a tackifier, a crosslinking agent, a stabilizer, a compatibilizer, an extruding aid, a filler, a pigment, a dye, a swelling agent, a chain transfer agent, and combinations thereof.

The present invention also provides a wound dressing that includes an apertured liquid permeable substrate and an absorbent, nonadherent polymer composition. The composition includes: a hydrophobic organic polymer matrix including a styrene-isoprene-styrene copolymer, a styrene-butadiene-styrene copolymer, or mixtures thereof; a plasticizing agent; and hydrophilic microparticles including an amine-containing organic polymer.

The present invention also provides a wound dressing that includes an apertured liquid permeable substrate and an absorbent, nonadherent polymer composition. The composition includes: a hydrophobic organic polymer matrix including a styrene-isoprene-styrene copolymer, a styrene-butadiene-styrene copolymer, or mixtures thereof; a plasticizing agent; and hydrophilic microparticles including a sodium polyacrylate copolymer.

The wound dressing of the present invention can be used to treat a wound.

As used herein, "a," "an," "the," "at least one," and "one or more" are used interchangeably. Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments.

The present invention provides wound dressings that include an apertured liquid permeable substrate and a polymer composition, which can be coated on or impregnated in the substrate. The polymer composition is absorbent and nonadherent and includes a hydrophobic organic polymer matrix, a plasticizer, and hydrophilic organic microparticles.

In the context of the polymer composition, the term "absorbent" means that the composition demonstrates a saline absorbency that is at least 50% of the dry weight of the polymer composition.

In the context of the polymer composition, the term "nonadherent" means that a composition used in the present invention coated on a substrate displays a 180° peel strength of less than 1 Newton per centimeter (N/cm) from stainless steel according the to test procedure described in the Examples Section. Preferably, the compositions used in the present invention do not adhere significantly to wound tissue such that they do not cause pain and/or destruction of the wound tissue upon removal. Although the composition itself is nonadherent, it should be understood that an adhesive (e.g., a pressure sensitive adhesive) could be added to an article that includes the composition, if desired.

Typically, the hydrophobic organic polymer matrix forms a continuous matrix with the hydrophilic particles substantially uniformly dispersed therein. This dispersion is often referred to as a hydrocolloid. The hydrophobic organic polymer matrix contributes significantly to the nonadherency of the polymer composition, whereas the hydrophilic organic microparticles contribute significantly to the absorbency.

The hydrophilic microparticles can be prepared from a wide range of polymers, including anionic, cationic, amphoteric, non-ionic polymers, or combinations thereof. In a preferred embodiment, the hydrophilic microparticles include an amine-containing polymer, which is more preferably a cationic quaternary ammonium salt of an organic polymer. In another preferred embodiment, the hydrophilic microparticles include an anionic polyacrylate.

The compositions used in the present invention are preferably light stable. By this it is meant that the compositions are stable to at least one of the following types of radiation: visible light; ultraviolet light; electron beam; and gamma ray sterilization.

As stated above, the polymer compositions used in the present invention are absorbent. Wound dressings containing such compositions used in the present invention can be used in their hydrated or swollen forms if desired. However, because the wound dressings include an apertured, liquid permeable substrate, the construction is prepared in such a way that the polymer composition can absorb fluid, yet in the swollen state, the apertures are not swollen shut. This allows fluid to traverse the dressing (perhaps into an overlying sorbent material, such as gauze) and not get trapped under it.

### HYDROPHOBIC ORGANIC POLYMER MATRIX

The polymer compositions include a hydrophobic organic polymer matrix. In this context, "hydrophobic" means that the polymer matrix is antagonistic to, sheds, tends not to combine with, or is incapable of dissolving in water. Hydrophobic materials are particularly desirable for nonadherent compositions and articles.

Examples of hydrophobic materials include, but are not limited to, polyisobutylene, polyethylene-propylene rubber, polyethylene-propylene diene-modified rubber, polyisoprene, styrene-isoprene-styrene, styrene-butadiene-styrene, styrene-ethylene-propylene-styrene, and styrene-ethylene-butylene-styrene. Particularly preferred hydrophobic materials include a styrene-isoprene-styrene copolymer and/or a styrene-butadiene-styrene copolymer, and even more preferred materials include a styrene-isoprene-styrene copolymer.

Other polymers (referred to herein as "optional secondary polymers") may also be included within the hydrophobic polymer matrix. The following are examples of such polymers.

Elastomeric polymers useful as optional secondary polymers in the invention are typically materials that form one phase at 21 °C, have a glass transition temperature less than 0°C, and exhibit elastomeric properties. The elastomeric polymers include, but are not limited to, polyisoprenes, styrene-diene block copolymers, natural rubber, polyurethanes, polyether-block-amides, poly-alpha-olefins, (C1-C20)acrylic esters of meth(acrylic) acid, ethylene-octene copolymers, and combinations thereof. Elastomeric materials useful in the present invention include, for example, natural rubbers such as CV-60 (a controlled viscosity grade natural rubber having Mooney viscosity of 60 +/- 5 ML, 1+4 at 100°C, available as an International commodity); butyl rubbers, such as Exxon Butyl 268 available from Exxon Chemical Co., Houston, Texas; synthetic poly-isoprenes such as CARIFLEX IR309, available from Kraton Polymers, Houston, Texas, and NATSYN 2210, available from Goodyear Tire and Rubber Co., Akron, Ohio; ethylene-propylene copolymers; polybutadienes; polyisobutylenes such as VISTANEX MM L-80, available from ExxonMobil Chemical Co.; and styrene-butadiene random copolymer rubbers such as AMERIPOL 1011A, available from BF Goodrich of Akron, Ohio.

Thermoplastic polymers useful as optional secondary polymers in the invention include, for example, polyolefins such as isotactic polypropylene; low density or linear low density polyethylene; medium density polyethylene; high density polyethylene; polybutylene; polyolefin copolymers or terpolymers, such as ethylene/propylene copolymer and blends thereof; ethylene-vinyl acetate copolymers such as ELVAX 260, available from E.I. DuPont de Nemours & Co., Wilmington, Delaware; ethylene acrylic acid copolymers; ethylene methacrylic acid copolymers such as SURLYN 1702, available from E. I. DuPont de Nemours & Co.; polymethylmethacrylate; polystyrene; ethylene vinyl alcohol; polyester; amorphous polyester; polyamides; fluorinated thermoplastics such a polyvinylidene fluoride; polytetrafluoroethylene; fluorinated ethylene/propylene copolymers; halogenated thermoplastics such as a chlorinated polyethylene; and combinations thereof. Other exemplary thermoplastic polymers are disclosed in International Publication No. WO 97/23577. Preferably, the thermoplastic polymer is a polyolefin.

Thermoplastic elastomeric polymers useful as optional secondary polymers in the invention are typically materials that form at least two phases at 21°C, flow at a temperature greater than 50°C and exhibit elastomeric properties. Thermoplastic elastomeric materials useful in the present invention include, for example, linear, radial, star and tapered styrene-isoprene block copolymers such as KRATON D1107P, available from Kraton Polymers, and EUROPRENE SOL TE 9110, available from EniChem Elastomers Americas, Inc. Houston, Texas, linear styrene-(ethylene/butylene) block copolymers such as KRATON G1657 available from Kraton Polymers, linear styrene-(ethylene/propylene) block copolymers such as KRATON G1657X available from Kraton Polymers, styrene-isoprene-styrene block copolymers such as KRATON D1119P available from Kraton Polymers, linear, radial, and star styrene-butadiene block copolymers such as KRATON D1118X, available from Kraton Polymers, and EUROPRENE SOL TE 6205 available from EniChem Elastomers Americas, Inc., polyetheresters such as HYTREL G3548, available from E. I. DuPont de Nemours & Co., and poly-alpha-olefin based thermoplastic elastomeric materials such as those represented by the formula -(CH₂-CHR) where R is an alkyl group containing 2 to 10 carbon atoms and poly-alpha-olefins based on metallocene catalysis such as ENGAGE EG8200, an ethylene/1-octene copolymer available from DuPont Dow Elastomers Co., Wilmington, Delaware. Other exemplary thermoplastic elastomers are disclosed in International Publication No. WO 96/25469.

Various combinations of optional secondary organic polymers in various amounts can be used to produce desired effects. This can be readily determined by one of skill in the art based on the teachings herein.

### ABSORBENT HYDROPHILIC MICROPARTICLES

The hydrophilic microparticles can include anionic, cationic, amphoteric, non-ionic polymers, or combinations thereof. Typically, the type and amount of microparticles are selected to provide the desired absorbency to the polymer composition used in the present invention.

Preferably, the microparticles, when in a substantially nonhydrated form, have an average particle size of 10 µm or less, and more preferably, 1 µm or less. Typically and preferably, the microparticles have an average particle size of 0.5 µm or more when in a substantially nonhydrated form.

Preferably, the hydrophilic polymer has a weight average molecular weight of at least 1000.

Preferably, the polymer is also dermatologically acceptable and non-reactive with the skin of the patient or with other components of the composition including any antimicrobial agents that may be present in therein.

Hydrophilic microparticles useful in the present invention may be made from a wide variety of synthetically prepared polymers, naturally occurring polymers, or chemically modified naturally occurring hydrophilic polymers. Varieties of polymers that can be used include synthetic polymers prepared from single or multiple monomers. The microparticles can be in an emulsion, such as an inverse emulsion that includes absorbent hydrophilic microparticles. In certain embodiments, the microparticles can be in a dispersion.

Non-limiting examples of such polymers include: polyhydroxyalkyl acrylates and methacrylates (e.g., those prepared from 2-hydroxyethyl acrylate, 2-hydroxyethyl methacrylate, 2-hydroxypropyl acrylate, 2-hydroxypropyl methacrylate, 2,3-dihydroxypropyl methacrylate); poly(meth)acrylic acid and salts thereof (wherein (meth)acrylic acid refers to methacrylic acid and acrylic acid); polyvinyl lactams (e.g., those prepared from N-vinyl lactams such as N-vinyl-2-pyrrolidone, 5-methyl-N-vinyl-2-pyrrolidone, 5-ethyl-N-vinyl-2-pyrrolidone, 3,3-dimethyl-N-vinyl-2-pyrrolidone, 3-methyl-N-vinyl-2-pyrrolidone, 3-ethyl-N-vinyl-2-pyrrolidone, 4-methyl-N-vinyl-2-pyrrolidone, 4-ethyl-N-vinyl-2-pyrrolidone, N-vinyl-2-valerolactam, and N-vinyl-2-caprolactam); polyvinyl alcohols; polyoxyalkylenes; polyacrylamides; polystyrene sulfonates, natural or synthetically modified polysaccarides (e.g., starch, glycogen, hemicelluloses, pentosans, gelatin, celluloses, pectin, chitosan, and chitin), alginates, gums (e.g., Locust Bean, Guar, Agar, Carrageenan, Xanthan, Karaya, alginates, tragacanth, Ghatti, and Furcelleran gums), cellulosics (e.g., those prepared from methyl cellulose, hydroxypropyl methyl cellulose, carboxymethyl cellulose, and hydroxypropyl cellulose); polymers prepared from water soluble amides (e.g., N-(hydroxymethyl)acrylamide and N-methacrylamide, N-(3-hydroxpropyl)acrylamide, N-(2-hydroxyethyl) methacrylamide, N-(1,1-dimethyl-3-oxabutyl)acrylamide N-[2-(dimethylamine)ethyl]acrylamide and -methacrylamide, N-[3-(dimethylamino)-2-hydroxylpropyl]methacrylamide, and N-[1,1-dimethyl-2-(hydroxymethyl)-3-oxabutyl]acrylamide)); polymers prepared from water-soluble hydrazine derivatives (e.g., trialkylamine methacrylimide, and dimethyl-(2-hydroxypropyl)amine methacrylimide); polymers prepared from mono-olefinic sulfonic acids and their salts, (such as sodium ethylene sulfonate, sodium styrene sulfonate and 2-acrylamideo-2-methylpropanesulfonic acid)). Other polymer include those prepared from the following monomers containing nitrogen in the non-cyclic or cyclic backbone of the monomer: 1-vinyl-imidazole, 1-vinyl-indole, 2-vinyl imidazole, 4(5)-vinyl-imidazole, 2-vinyl-1-methyl-imidazole, 5-vinyl-pyrazoline, 3-methyl-5-isopropenyl-pyrazole, 5-methylene-hydantoin, 3-vinyl-2-oxazolidone, 3-methacrylyl-2-oxazolidone, 3-methacrylyl-5-methyl-2-oxazolidone, 3-vinyl-5-methyl-2-oxazolidone, 2- and 4-vinyl-pyridine, 5-vinyl-2-methyl-pyridine, 2-vinyl-pyridine-1-oxide, 3-isopropenyl-pyridine, 2- and 4-vinyl-piperidine, 2- and 4-vinyl-quinoline, 2,4-dimethyl-6-vinyl-s-triazine, and 4-acrylyl-morpholine.

For certain embodiments, the microparticles are prepared from amine-containing organic polymers. Preferably, the amine-containing hydrophilic polymer include a quaternary amine, and more preferably, the amine-containing polymer is a quaternary ammonium salt of an organic polymer. Examples include, but are not limited to, polymerization products of cationic vinyl monomers as disclosed in EP 0 489 967 A1, and inherently antimicrobial quaternary amine polymers as described in U.S. Pat. No. 6,039,940.

For certain embodiments, the microparticles are prepared from carboxylic acid-containing organic polymers. Examples of such microparticles include sodium polyacrylate (i.e., a copolymer of sodium acrylate and acrylic acid) microparticles such as those commercially available under the trade designation SALCARE SC91 from Ciba Specialty Chemicals (High Point, NC).

Preferred microparticles are described in EP 172 724 A2 and EP 126 528 A2 made by reverse phase polymerization and have a dry particle size below 4 µm

Other suitable polymeric microparticles can be prepared from a quaternary ammonium monomer, which is a salt having an organo-ammonium group and a monoethylenically unsaturated group. For certain embodiments, the quaternary ammonium monomer has the following general Formula (I): 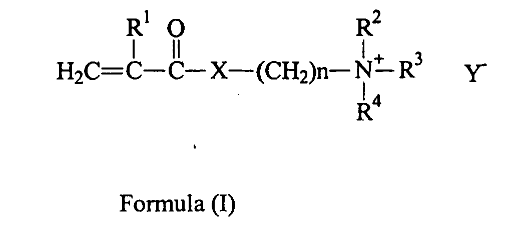 wherein: n is 2 to 10, preferably 2 to 3; R¹ is H or CH₃; R², R³, and R⁴ are each independently linear or branched organic groups, preferably having 1 to 16 carbon atoms (on average); X is O or NH; and Y⁻ is an acceptable anionic counterion to the N⁺ of the quaternary ammonium group (e.g., one that does not adversely affect the polymerization of the monomers or antimicrobial activity of an added antimicrobial agent).

Preferably, R², R³, and R⁴ are each independently alkyl, aryl, alkaryl, or aralkyl groups. Alkyl groups are preferably lower alkyl, having 1 to 16 carbon atoms (on average) with methyl and ethyl groups being particularly preferred. Aryl is preferably phenyl but can be any suitable aromatic moiety such as those selected from the group consisting of phenyl, thiophenyl, naphthyl, biphenyl, pyridyl, pyrimidinyl, pyrazyl, pyridazinyl, furyl, thienyl, pyrryl, quinolinyl, bipyridyl, and the like. Representative of an aralkyl grouping is benzyl and representative of an alkaryl grouping is tolyl. X is preferably O. Representative counterions (Y') are Cl⁻, Br⁻, HSO₄⁻, CH₃CH₂OSO₃⁻, and CH₃OSO₃⁻, with the chloride salts being particularly preferred. Alkyl groups can be straight or branched chain and alkyl and aryl groups can be substituted by non-interfering substituents that do not obstruct with the functionality of the polymers.

Useful copolymerizable quaternary ammonium monomers include, but are not limited to, those selected from 2-(meth)acryloxyethyl trialkyl ammonium halides and sulfates, and mixtures thereof. Examples of such compounds include, but are not limited to, 2-(meth)acryloxyethyl trimethyl ammonium chloride, CH₂=C(H or CH₃)CO₂CH₂CH₂N(CH₃)₃Cl; 2-(meth)acryloxyethyl trimethyl ammonium methyl sulfate, CH₂=C(H or CH₃)CO₂CH₂CH₂N(CH₃)₃OSO₂OCH₃; 2-(meth)acryloxyethyl methyl diethyl ammonium methyl sulfate, CH₂=C(H or CH₃)CO₂CH₂CH₂N(CH₃)(C₂H₅)₂OSO₂OCH₃; 2-(meth)acryloxyethyl dimethyl benzyl ammonium chloride, CH₂=C(H or CH₃)CO₂CH₂CH₂N(CH₃)₂(C₆H₅CH₂)Cl (all of the preceding monomers available from Ciba Specialty Chemicals, Woodbridge, NJ); 2-(methylacryloxy)ethyl dimethyl hexadecyl ammonium bromide, CH₂=C(CH₃)CO₂CH₂CH₂N(CH₃)₂(C₁₆H₃₃)Br (described in U.S. Pat. No. 5,437,932 (Ali et al.)); and the like. Various combinations of these monomers can be used if desired. Due to their availability, effectiveness in reinforcing (meth)acrylate polymers and their antimicrobial activity, particularly preferred quaternary ammonium monomers are 2-acryloxyethyl trimethyl ammonium methyl sulfate and 2-acryloxyethyl methyl diethyl ammonium methyl sulfate. Such monomers are typically hydrophilic. Various combinations of other monoethylenically unsaturated monomers that are reinforcing monomers can be used in the polymers used in the present invention. Such reinforcing monomers include, but are not limited to, acrylic acid, methacrylic acid, ethylene vinyl acetate, and N,N-dimethylacrylamide.

As an alternative approach to providing polymers that contain a quaternary ammonium functional unit, it is possible to start with an amine monomer and form the quaternary ammonium unit following polymerization. For certain embodiments, the amine monomers have the following general Formula (II): 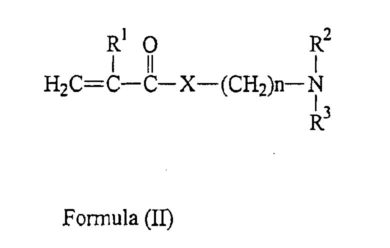 wherein n, R¹, R², R³, and X are the same as defined for Formula (I).

As stated above, the microparticles can be in an emulsion, such as an inverse emulsion. One type of inverse emulsion can be defined as a continuous hydrophobic liquid phase (e.g., mineral oil) and hydrophilic polymer particles dispersed within the hydrophobic liquid phase. Suitable examples of such materials are described in EP 0 126 528 A2. Such a material is commercially available under the trade designation SALCARE from Ciba Specialty Chemicals (High Point, NC). Suitable examples include SALCARE 95 and 96 which include a cationic homopolymer of the methyl chloride quaternary salt of 2-(dimethylamino)ethyl methacrylate (CAS No. 26161-33-1).

Other amine-containing polymers can be made from amine-containing monomers as described below and in EP 0 489 967 A1 and U.S. Pat. No. 6,039,940.

Monomers can be polymerized using techniques such as solution polymerization, emulsion polymerization, bulk polymerization, suspension polymerization, and the like. In particular, emulsion polymerization and suspension polymerization are preferable because the molecular weight of the polymer becomes high; solution polymerization is preferable because the molecular weight distribution is comparatively narrow; and bulk polymerization is favorable because no solvent is used.

In such polymerizations, initiators can be used to generate free-radicals upon the application of activating energy such as those conventionally used in the polymerization of ethylenically unsaturated monomers. Included among useful free-radical initiators are the thermally activated initiators such as organic peroxides, organic hydroperoxides, and azo-compounds. Representative examples of such initiators include, but are not limited to, benzoyl peroxide, tertiary-butyl perbenzoate, diisopropyl peroxydicarbonate, cumene hydroperoxide, azobis(isobutyronitrile), and the like. Generally, the thermal initiators are typically used in amounts from 0.01 to 5 percent by weight of monomer.

The polymerization of the polymer may also be initiated by photoinitiators. Such photochemically activated initiators are well known and have been described in the polymerization art; e.g., Chapter II of "Photochemistry" by Calvert and Pitts, John Wiley and Sons (1966) and in Progress in Organic Coatings, 13, 123-150 (1985). Representative examples of such initiators include benzoin, benzoin methyl ether, benzoin isopropyl ether, benzoin isobutyl ether, and 2-hydroxy-2-methyl-1-phenyl-1-propane, benzildimethylketal and benzildiethylketal, 2-hydroxy-1-(4-(2-hydroxyethoxy)phenyl)-2-methyl-1-propanone. A presently preferred photoinitiator is 2-hydroxy-1-(4-(2-hydroxyethoxy)phenyl)-2-methyl-1-propanone. Generally, photoinitiators are used in amounts from 0.01 to 5 percent by weight of monomer.

The polymerization of the polymer may also be initiated by electromagnetic radiation such as electron beams and the gamma-rays of cobalt 60, and the like. The irradiation dose is typically between 1 and 100 kGy.

The polymer may be crosslinked by adding a crosslinking compound or through electron beam or gamma radiation. A crosslinking compound can be a multi-ethylenically unsaturated compound wherein the ethylenic groups are vinyl groups, allyl groups, and/or methallyl groups bonded to nitrogen or oxygen atoms. Exemplary compounds include divinyl, diallyl or dimethallyl esters (e.g., divinyl succinate, divinyl adipate, divinyl maleate, divinyl oxalate, divinyl malonate, divinyl glutarate, diallyl itaconate, diallyl maleate, diallyl fumarate, diallyl diglycolate, diallyl oxalate, diallyl adipate, diallyl succinate, diallyl azelate, diallyl malonate, diallyl glutarate, dimethallyl maleate, dimethallyl oxalate, dimethallyl malonate, dimethallyl succinate, dimethallyl glutarate, and dimethallyl adipate), divinyl, diallyl or dimethallyl ethers (e.g., diethyleneglycol divinyl ether, butanediol divinyl ether, ethylene glycol divinyl ether, ethylene glycol diallyl ether, diethylene glycol diallyl ether, butane diol diallyl ether, ethylene glycol dimethallyl ether, diethylene glycol dimethallyl ether, and butane diol dimethallyl ether), divinyl, diallyl or dimethallyl amides including bis(N-vinyl lactams), (e.g., 3,3'-ethylidene bis(N-vinyl-2-pyrrolidone)), and divinyl, diallyl or dimethallyl ureas.

### PLASTICIZING AGENTS

Plasticizing agents (i.e., plasticizers) selected for use in the compositions used in the present invention can possess a range of properties. Generally, the plasticizing agents can be liquid, semi-solid or solid, have a range of molecular weights and architectures (e.g., be monomeric or polymeric in nature), and are compatible with the other components of the polymer composition. Additionally, mixtures of solid and liquid, monomeric and polymeric and other combinations of plasticizing agents can be used in the present invention.

For certain embodiments, elastomeric plasticizing agents can be used. Such plasticizing agents can be derived from low molecular weight naphthalenic oils, or low molecular weight acids, or alcohols, which are then esterified with respectively a monofunctional alcohol or monofunctional acid. Examples of these are mineral oil, cetostearyl alcohol, cetyl alcohol, cholesterol, coconut oil, oleyl alcohol, steryl alcohol, and squalane. Some elastomers are more compatible with esters of mono- and multibasic acids, such as isopropyl myristate, isopropyl palmitate, dibutyl phthalate, diisoctyl phthalate, dibutyl adipate, dibutyl sebacate, and the like. Useful polymeric plasticizing agents include non-acrylic plasticizing agents, which are typically derived from cationically or free-radically polymerizable monomers, condensation polymerizable monomers, or ring-opening polymerizable monomers to make low molecular weight polymers. Examples of these polymeric plasticizing agents include materials such as polyurethanes, polyureas, polyvinylethers, polyethers, polyesters, and the like.

Useful plasticizing agents are compatible with the polymer(s) of the hydrophobic polymer matrix, such that once the plasticizing agent is mixed with therein, the plasticizing agent does not phase separate from the hydrophobic polymer matrix. By "phase separation" or "phase separate", it is meant that by differential scanning calorimetry (DSC) no detectable thermal transition, such as a melting or glass transition temperature can be found for the pure plasticizing agent in the plasticized composition. Some migration of the plasticizing agent from or throughout the plasticized composition can be tolerated, such as minor separation due to composition equilibrium or temperature influences, but the plasticizing agent does not migrate to the extent of phase separation between the polymer(s) of the hydrophobic polymer matrix and the plasticizing agent.

Preferably, useful plasticizing agents are non-reactive, thus preventing copolymerization with the reactive groups of the polymers in the hydrophobic polymer matrix of the hydrophilic microparticles. Thus, for example, plasticizing agents having acrylate functionality, methacrylate functionality, styrene functionality, or other ethylenically unsaturated, free radically reactive functional groups are generally not used.

Generally, liquid plasticizing agents are readily compoundable with hydrophobic polymer matrix that includes one or more elastomers using an extruder. In addition, liquid plasticizing agents may be delivered directly to a tacky elastomer, if used in the composition, in order to make it less tacky or non-tacky.

Although somewhat more challenging to use, semi-solid (such as petrolatum) and solid plasticizing agents (such as paraffin wax, beeswax, microcrystalline wax, cetyl esters wax) can advantageously be used in compositions used in the present invention where the controlled plasticization is desired. For example, hot melt processible compositions can be easily transported and handled prior to melt compounding if the hydrophobic polymer matrix and the plasticizing agent components are solid and non-tacky. Once heated to the melting or glass transition temperature of the solid plasticizing agent, the polymer of the matrix is plasticized.

The plasticizing agent is typically used in amounts of from about 1 to 2000 parts by weight per 100 parts of the hydrophobic polymer.

### OPTIONAL BIOACTIVE AGENTS

The polymer compositions used in the present invention can optionally include a bioactive agent. Typically, the bioactive agents are antimicrobial (e.g., antibacterial or antifungal) agents. Such actives are capable of destroying microbes, preventing the development of microbes or preventing the pathogenic action of microbes. An effective amount of a bioactive agent may be added to the present compositions. If use, this amount is typically at least 0.001%, based on the total weight of the composition.

Examples include, but are not limited to, beta-lactam drugs, quinolone drugs, ciprofloxacin, norfloxacin, tetracycline, erythromycin, amikacin, 2,4,4'-trichloro-2'-hydroxy diphenyl ether, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, miconazole, ketaconazole, amanfadine hydrochloride, amanfadine sulfate, octopirox, parachlorometa xylenol, nystatin, tolnaftate, pyrithiones (especially zinc pyrithione which is also known as ZPT), dimethyldimethylol hydantoin, methylchloroisothiazolinone/methylisothiazolinone, sodium sulfite, sodium bisulfite, imidazolidinyl urea, diazolidinyl urea, benzyl alcohol, 2-bromo-2-nitropropane-1,3-diol, formalin (formaldehyde), iodopropenyl butylcarbamate, chloroacetamide, methanamine, methyldibromonitrile glutaronitrile (1,2-dibromo-2,4-dicyanobutane), glutaraldehyde, 5-bromo-5-nitro- 1,3-dioxane, phenethyl alcohol, o-phenylphenol/sodium o-phenylphenol, sodium hydroxymethylglycinate, polymethoxy bicyclic oxazolidine, dimethoxane, thimersal dichlorobenzyl alcohol, captan, chlorphenenesin, dichlorophene, chlorobutanol, glyceryl laurate, halogenated diphenyl ethers like 2,4,4'-trichloro-2'-hydroxy-diphenyl ether, 2,2'-dihydroxy-5,5'-dibromo-diphenyl ether, phenolic compounds like phenol, 2-methyl phenol, 3-methyl phenol, 4-methyl phenol, 4-ethyl phenol, 2,4-dimethyl phenol, 2,5-dimethyl phenol, 3,4-dimethyl phenol, 2,6-dimethyl phenol, 4-n-propyl phenol, 4-n-butyl phenol, 4-n-amyl phenol, 4-tert-amyl phenol, 4-n-hexyl phenol, 4-n-heptyl phenol, mono- and poly-alkyl and aromatic halophenols such as p-chlorophenol, methyl p-chlorophenol, ethyl p-chlorophenol, n-propyl p-chlorophenol, n-butyl p-chlorophenol, n-amyl p-chlorophenol, sec-amyl pchlorophenol, n-hexyl p-chlorophenol, cyclohexyl p-chlorophenol, n-heptyl p-chlorophenol, n-octyl p-chlorophenol, o-chlorophenol, methyl o-chlorophenol, ethyl o-chlorophenol, n-propyl ochlorophenol, n-butyl o-chlorophenol, n-amyl o-chlorophenol, tert-amyl o-chlorophenol, n-hexyl o-chlorophenol, n-heptyl o-chlorophenol, o-benzyl p-chlorophenol, o-benzyl-m-methyl p-chlorophenol, o-benzyl-m, m-dimethyl p-chlorophenol, o-phenylethyl p-chlorophenol, o-phenylethyl-m-methyl p-chlorophenol, 3-methyl p-chlorophenol, 3,5-dimethyl p-chlorophenol, 6-ethyl-3-methyl p-chlorophenol, 6-n-propyl-3-methyl p-chlorophenol, 6-iso-propyl-3-methyl p-chlorophenol, 2-ethyl-3,5-dimethyl p-chlorophenol, 6-sec-butyl-3-methyl p-chlorophenol, 2-iso-propyl-3,5-dimethyl pchlorophenol, 6-diethylmethyl-3-methyl p-chlorophenol, 6-iso-propyl-2-ethyl-3-methyl p-chlorophenol, 2-sec-amyl-3,5-dimethyl p-chlorophenol 2-diethylmethyl-3,5-dimethyl p-chlorophenol, 6-sec-octyl-3-methyl p-chlorophenol, p-chloro-m-cresol, p-bromophenol, methyl pbromophenol, ethyl p-bromophenol, n-propyl p-bromophenol, n-butyl p-bromophenol, n-amyl p-bromophenol, sec-amyl p-bromophenol, n-hexyl p-bromophenol, cyclohexyl p-bromophenol, o-bromophenol, tert-amyl o-bromophenol, n-hexyl o-bromophenol, n-propyl-m,m-dimethyl o-bromophenol, 2-phenyl phenol, 4-chloro-2-methyl phenol, 4-chloro-3-methyl phenol, 4-chloro-3,5-dimethyl phenol, 2,4-dichloro-3,5-dimethylphenol, 3,4,5,6-tetrabromo-2-methylphenol, 5-methyl-2-pentylphenol, 4-isopropyl-3-methylphenol, para-chloro-meta-xylenol (PCMX), chlorothymol, 5-chloro-2-hydroxydiphenylmethane, resorcinol and its derivatives including methyl resorcinol, ethyl resorcinol, n-propyl resorcinol, n-butyl resorcinol, n-amyl resorcinol, n-hexyl resorcinol, n-heptyl resorcinol, n-octyl resorcinol, n-nonyl resorcinol, phenyl resorcinol, benyl resorcinol, phenylethyl resorcinol, phenylpropyl resorcinol, p-chlorobenzyl resorcinol, 5-chloro 2,4-dihydroxydiphenyl methane, 4'-chloro 2,4-dihydroxydiphenyl methane, 5-bromo 2,4-dihydroxydiphenyl methane, and 4'-bromo 2,4-dihydroxydiphenyl methane, bisphenolic compounds like 2,2'-methylene bis (4-chlorophenol), 2,2'-methylene bis (3,4,6-trichlorophenol), 2,2'-methylene bis (4-chloro-6-bromophenol), bis (2-hydroxy-3,5-dichlorophenyl) sulphide, and bis (2-hydroxy-5-chlorobenzyl)sulphide, benzoic esters (parabens) like methylparaben, propylparaben, butylparaben, ethylparaben, isopropylparaben, isobutylparaben, benzylparaben, sodium methylparaben, and sodium propylparaben, halogenated carbanilides (e.g., 3,4,4'-trichlorocarbanilides), 3-trifluoromethyl-4,4'-dichlorocarbanilide, 3,3',4-trichlorocarbanilide, etc.), cationic actives such as benzalkonium chloride, and clotrimazole.

Another class of antimicrobial agents (i.e., actives), which are useful in the present invention, are the so-called "natural" antibacterial actives, referred to as natural essential oils. These actives derive their names from their natural occurrence in plants. Typical natural essential oil antibacterial actives include oils of anise, lemon, orange, rosemary, wintergreen, thyme, lavender, cloves, hops, tea tree, citronella, wheat, barley, lemongrass, grapefruit seed, cedar leaf, cedarwood, cinnamon, fleagrass, geranium, sandalwood, violet, cranberry, eucalyptus, vervain, peppermint, gum benzoin, basil, fennel, fir, balsam, menthol, ocmea origanum, Hydastis carradensis, Berberidaceae daceae, Ratanhiae and Curcuma longa. Also included in this class of natural essential oils are the key chemical components of the plant oils, which have been found to provide the antimicrobial benefit. These chemicals include, but are not limited to, anethol, catechole, camphene, thymol, eugenol, eucalyptol, ferulic acid, farnesol, hinokitiol, tropolone, limonene, menthol, methyl salicylate, carvao-ol, terpineol, verbenone, berberine, ratanhiae extract, caryophellene oxide, citronellic acid, curcumin, nerolidol and geraniol.

The bioactive agent can be present in the polymer composition in an amount to produce a desired effect (e.g., antimicrobial effect).

### OTHER OPTIONAL ADDITIVES

The polymer compositions used in the present invention can include a wide variety of optional additives. Examples include, but are not limited to, secondary bioactive agents, swelling agents, fillers, pigments, dyes, tackifiers, crosslinking agents, stabilizers, compatibilizers, extruding aids, chain transfer agents, and combinations thereof.

In certain embodiments, polymer compositions used in the present invention can include fillers, which can be inorganic or organic. Examples of inorganic fillers include, but are not limited to, barytes, chalk, gypsum, kieserite, sodium carbonate, titanium dioxide, cerium oxide, silica dioxide, kaolin, carbon black, and hollow glass microbeads. Examples of organic fillers include, but are not limited to, powders based on polystyrene, polyvinyl chloride, urea-formaldehyde, and polyethylene. The fillers may be in the form of fibers, such as chopped fibers. Examples of suitable chopped fibers include glass fibers (typically 0.1 millimeter (mm) to 1 mm long) or fibers of organic origin such as, for example, polyester or polyamide fibers.

In order to confer color to the polymer compositions it is possible to use dyes or colored pigments of an organic or inorganic basis such as, for example, iron oxide or chromium oxide pigments or phthalocyanine- or monoazo-based pigments.

### METHODS OF PREPARATION OF POLYMER COMPOSITIONS AND ARTICLES

For certain embodiments, the components are combined in a manner to produce a polymer composition wherein at least a portion of the bioactive agent, if used, is incorporated within microparticles. Preferably, this results from combining the components by hot mixing without a solvent (so-called hot-melt process), by blending an elastomer with an oily plasticizer and antioxidants, and then by adding a hydrocolloid either as finely divided powder or as an inverse emulsion. If active agents are provided, these may be added to either the elastomer or the hydrocolloid.

In certain embodiments, an inverse emulsion that includes hydrophilic organic microparticles is combined with water and a bioactive agent under conditions effective to distribute (preferably, dissolve) at least a portion of the bioactive agent in the hydrophilic organic microparticles. Optionally, a secondary organic polymer can be added to the mixture of the inverse emulsion, solvent, and an optional bioactive agent. Once sufficiently mixed to impregnate at least a portion of the bioactive agent, if used, into the hydrophilic particles, the solvent is removed, if desired.

In other embodiments, monomers for a hydrophilic organic polymer are combined with an optional bioactive agent under conditions effective to polymerize the monomers and distribute (preferably dissolve) at least a portion of the bioactive agent, if used, in the hydrophilic organic polymer. The bioactive agent, if used, can be present during the polymerization process or added after the polymerization is complete. Optionally, a secondary organic polymer can be added to the hydrophilic organic polymer with the bioactive agent, if used, distributed therein.

The polymer compositions, with or without the bioactive agent therein, can be melt processed (e.g., extruded or molded) or solvent cast to form the desired products (e.g., wound dressing).

The materials used to prepare the polymer compositions used in the present invention are melt processable if they are fluid or pumpable, and they do not significantly degrade or gel at the temperatures used to melt process (e.g., extruding or compounding) the composition (e.g., at least 50°C and up to 300°C). Preferably, such materials have a melt viscosity of at least 10 poise and often up to 1,000,000 poise, as measured by capillary melt rheometry at the processing temperatures and shear rates employed in extrusion. Typically, suitable materials possess a melt viscosity within this range at a temperature of at least 175°C and often up to 225°C and a shear rate of 100 seconds⁻¹.

Continuous melt process forming methods include drawing the extruded composition out of a film die and subsequently contacting a moving plastic web or other suitable backing. Another continuous forming method involves directly contacting the extruded composition to a rapidly moving plastic web or other suitable substrate. In this method, the extruded composition can be applied to a moving web using a die having flexible die lips such a reverse orifice coating die and other contact dies using rotating rods. The composition can also be extruded in the form of continuous fibers and blown micro-fiber webs as disclosed in Wente, Van A., "Superfine Thermoplastic Fibers", Industrial Engineering Chemistry, Vol. 48, pp. 1342-1346; Wente, Van A. et al., "Manufacture of Superfine Organic Fibers", Report No. 4364 of the Naval Research Laboratories, published May 25, 1954; and U.S. Pat. Nos. 5,176,952 and 3,841,953. After melt process forming the composition is solidified by quenching using either direct methods, such as chill rolls or water baths, or indirect methods, such as air or gas impingement, or both.

Articles can be prepared using compositions described herein according to a variety of methods, particularly coating methods. When a porous substrate is coated, the process of coating the porous substrate with the composition typically allows the yarns, filaments, or film to be properly trapped in the composition, while leaving most of the apertures unobstructed by the composition. Depending on the structure of the support used, the amount of composition employed will vary over a wide range (typically from 50 grams per square meter (g/m²) to 300 g/m², and preferably from 60 g/m² to 160 g/m²).

In certain embodiments, the coating can be carried out hot, without a solvent, using a continuous process in which the substrate is directed over a first coating roll covered with a layer of molten composition having a predetermined thickness, and then over a second roll which removes the composition lying within the apertures of the substrate. The substrate thus covered with gel only on the yarns, filaments, or film is then cooled in a stream of air so that the composition cannot flow and remains uniformly distributed around the yarns, filaments, or film. If necessary, a system producing a laminar stream of air is provided, which system is able both to correct the distribution of the composition around the yarns, filaments, or film and to unblock any substrate apertures, which would not have been open in the previous step of the process.

According to a variant of this process, a substrate can be passed through a bath of molten polymeric composition (for example, at a temperature of 120°C to 200°C). The substrate covered with molten composition is then passed between two fixed rolls pressed against each other with a predetermined gap, so as to remove the excess composition. The amount of composition remaining on the yarns, filaments, or film depends essentially on the gap set between the fixed rolls. The covered product is then cooled and treated in a manner similar to the previous process.

If desired, the cooled coated substrate can be covered with two protective films (for example, thin polyester films). These films may or may not require a nonstick treatment and can function to facilitate extraction from a package and in handling the article. If desired, the coated substrate can be cut into individual compresses, of sizes suitable for the use, packaged in sealed sachets, and sterilized.

Solvent casting may also be used to prepare the articles of the present invention. This method typically employs a common solvent, selected for compatibility with the polymer composition components. Such common solvents include, for example, toluene and tetrahydrofuran. Specific selection of a common solvent for a particular subset of the present invention is within the skill of the art. In the solvent casting method, the materials included in the composition are blended to form a uniform mixture, then coated onto a carrier web or a backing (described below) using a known coating technique such as curtain coating, die coating, knife coating, roll coating, or spray coating. A preferred coating method is knife coating. The solvent is then removed from the coated backing, usually with the aid of a drying oven for a time and temperature selected to remove any undesirable level of residual solvent.

Layered constructions can also be prepared using lamination, coating, or extrusion techniques known to one of skill in the art and as described, for example, in U.S. Pat. No. 6,379,791.

If desired, compositions used in the present invention can be sterilized. Methods of sterilization include treatment with electron beam or gamma radiation.

### WOUND DRESSINGS

The polymer compositions are used in wound dressings, i.e., medical articles that are applied directly to or contact a wound. Such articles include a backing (i.e., a support substrate) that is porous. The composition can be coated on the support substrate or impregnated into it, for example.

Suitable materials are preferably flexible, and may be fabric, non-woven or woven polymeric films, metallic, paper, and/or combinations thereof. More specifically, it is desirable to use a liquid permeable (e.g., with respect to moisture vapor), open apertured substrate (e.g., a scrim). For certain embodiments it is desirable to use an open- or closed-cell foam, such as that disclosed in U.S. Pat. Nos. 6,548,727 and 5,409,472.

The substrates (i.e., backings) are preferably porous to allow the passage of wound fluids, moisture vapor, and air. Hence, the porous substrates are liquid permeable.

Suitable porous substrates include knits, wovens (e.g., cheese cloth and gauze), nonwovens (including spun-bonded nonwovens), extruded porous sheets, and perforated sheets. The apertures (i.e., openings) in the porous substrates are of sufficient size and sufficient number to facilitate high breathability. For certain embodiments, the porous substrates have at least 1 aperture per square centimeter. For certain embodiments, the porous substrates have no greater than 225 apertures per square centimeter. For certain embodiments, the apertures have an average opening size (i.e., the largest dimension of the opening) of at least 0.1 millimeter (mm). For certain embodiments, the apertures have an average opening size (i.e., the largest dimension of the opening) of no greater than 0.5 cm.

For certain embodiments, the porous substrates have a basis weight of at least 5 grams/meter². For certain embodiments, the porous substrates have a basis weight of no greater than 200 grams/meter².

The porous substrates (i.e., backings) are preferably flexible yet resistant to tearing. For certain embodiments, the thickness of the porous substrates is at least 0.0125 mm. For certain embodiments, the thickness of the porous substrates is no greater than 3 mm.

The porous substrates may be opaque or translucent. Normally they have a skin color, but "designer" colors and patterns, as well as cartoon character designs, are becoming popular.

Materials of the backing or support substrate include a wide variety of materials including paper, natural or synthetic fibers, threads and yarns made from materials such as cotton, rayon, wool, hemp, jute, nylon, polyesters, polyacetates, polyacrylics, alginates, ethylene-propylene-diene rubbers, natural rubber, polyesters, polyisobutylenes, polyolefins (e.g., polypropylene polyethylene, ethylene propylene copolymers, and ethylene butylene copolymers), polyurethanes (including polyurethane foams), vinyls including polyvinylchloride and ethylene-vinyl acetate, polyamides, polystyrenes, fiberglass, ceramic fibers, and/or combinations thereof.

For particular purposes, the backing may be coated on one or both major surfaces, with a primer or a release agent, which may be a low-adhesion backsize (LAB) material. For example, when using a plasticized polyvinylchloride (PVC) backing, an embodiment of the present invention comprising a butadiene- or isoprene- containing polymer along with a polyisoprene-polyvinylpyridine (PI-PVP) compatibilizer has a particular advantage in that the composite PSA has an affinity for acidic PVC.

### EXAMPLES

Objects and advantages of this invention are further illustrated by the following examples, but the particular materials and amounts thereof recited in these examples, as well as other conditions and details, should not be construed to unduly limit this invention.

### Materials

KRATON D1124K- radial 4-arm star polystyrene-polyisoprene (SI)₄ thermoplastic elastomeric copolymer having 30 wt-% polystyrene, available from KRATON Polymers, Houston, Texas.

SALCARE SC95- sub-micron cationic inverse emulsion consisting of polymerized methylchloride quaternary ammonium salt of dimethylaminoethylmethacrylate (DMAEMA) microparticles dispersed in mineral oil and proprietary non-ionic surfactant, available from Ciba Specialty Chemicals, High Point, North Carolina.

SALCARE SC91 -sub-micron anionic inverse emulsion consisting of polymerized sodium acrylate copolymer microparticles dispersed in mineral oil and proprietary non-ionic surfactant, available from Ciba Specialty Chemicals, High Point, North Carolina.

KAYDOL -mineral oil available from Crompton Corporation, formerly Witco Corporation.

IRGANOX 1010 -Phenolic antioxidant available from Ciba Specialty Chemicals, Tarrytown, New York.

Polyester Knitted Fabric was a 24 mesh polyester knit (61 g/m²) purchased from Lamports Filter Media, Inc, Cleveland, OH.

### Peel Adhesion Test

Peel adhesion is measured as 180° peel from steel plates, at 23°C, 50% relative humidity (RH), 305 millimeters per minute (mm/min), 25 mm wide using a Model 3M90 Slip/Peel tester (IMASS, Inc., Accord, MA). The samples were conditioned for 24 hours at controlled temperature and humidity. After conditioning the samples were adhered to a stainless steel panel using 2 kilograms (kg) roller and 4 passes. The samples were peeled from the stainless steel plate after 15 minutes of dwell time using a 0.305 meter/minute (m/min) peel rate. Typically, two 0.13 meter (m) long samples were measured and the average peel force recorded in ounces/inch (oz/in) and converted to Newtons per decimeter (N/dm).

### Saline Absorbency Test

Samples (2.54 cm by 2.54 cm) were soaked in saline. The samples were removed from the saline at various times and were lightly dabbed with a paper towel. The weight was recorded and the samples were placed back into the saline solution. The weight of saline absorbed per weight of dry coating was calculated as a function of swelling time in the saline using the following equation: (weight saline absorbed)/(dry coating sample weight) = [(saline swollen weight) - (dry sample weight)]/[(dry sample weight) - (weight of substrate)].

### Preparation of Examples

Examples were prepared by first preparing a hydrophobic gel and then incorporating hydrophilic microparticles and a support substrate to make an article.

### Preparation of Gel

KRATON D1124K styrene-isoprene-styrene (SIS) pellets were gravimetrically fed into the feed throat (barrel section 1) of a Werner Pfleiderer ZSK30 co-rotating twin-screw extruder (TSE) having a 30 mm diameter and 15 barrel sections.

Each temperature zone was a combination of two barrel sections (e.g., Zone 1 corresponded to barrel sections 2 and 3). Barrel section 1 was controlled at full cooling capacity for all SIS gel lots. A powdered antioxidant (IRGANOX 1010) was also gravimetrically fed into barrel section 1. KAYDOL mineral oil was heated and added to the TSE as described in International Publication No. WO 97/00163. The disclosed compounding process provides a method for making a gel by melting of the SIS elastomer followed by addition of the heated mineral oil. Heated mineral oil was sequentially injected into barrel sections 4, 6, 8, 10 and 12, respectively. The TSE screw speed was controlled to 400 revolutions per minute (rpm). The TSE temperature profile was controlled to 204°C, 227°C, 227°C, 204°C, 182°C, 171°C, and 93°C for zones 1-7, respectively. The heated oil injections were controlled to 204°C, 204°C, 204°C, 177°C, and 177°C, respectively. Table 1 contains the material flow rates and Table 2 contains the compositional information for the SIS gel.

**Table 1. SIS gel lot flow rates**

| SIS (g/min) | Barrel Section(S) and Oil addition number and Rate (g/min) | | | | | Total KAYDOL Oil (g/min) | IRGANOX 1010 (g/min) | Total Flow Rate (g/min) |
|---|---|---|---|---|---|---|---|---|
| | S4 | S6 | S8 | S10 | S12 | | | |
| | Oil 1 | Oil 2 | Oil 3 | Oil 4 | Oil 5 | | | |
| 227 | 74 | 100 | 120 | 120 | 108 | 522 | 8 | 757 |

**Table 2. SIS gel composition**

| SIS Type | SIS (wt-%) | KAYDOL oil (wt-%) | IRGANOX 1010 (wt-%) |
|---|---|---|---|
| KRATON D1124K | 30.0 | 69.0 | 1.0 |

### Preparation of Dressing

Examples 1 and 2 were prepared by combining the pre-compounded SIS gel with SALCARE SC95 or SALCARE SC91 in a Haake 25 mm diameter, fully intermeshing counter-rotating TSE. Examples 1 and 2 were prepared by re-melting the SIS gel in a Bonnot extruder operating at 127°C. The molten gel was injected at 22.8 grams per minute into barrel section 2 of the TSE. SALCARE inverse emulsion was injected at ambient temperature into barrel section 4 at 15.2 grams per minute (g/min) using a Zenith gear pump. The TSE was controlled at 300 rpm screw speed and 121°C temperature. The total material throughput was 38.0 grams per minute. The SIS gel/SALCARE blend was discharged out of the TSE into a transport hose using a Zenith gear pump. A transport hose conveyed the molten gel blend to a 0.15meter (m) wide single orifice film die. The transport hose and die were both controlled to 121°C. The molten gel blend was extruded into a nip formed by two gapped and polished steel rolls controlled to 110°C. A polyester (PET) knitted fabric having 0.8 mm by 0.7 mm (0.56 mm²) rectangular open apertures, 0.20 millimeter (mm) thickness and 0.15 meter (m) width was also fed into the nip at 1.4 m/min speed. As the fabric exited the nip, the gel-coated article was cooled in air before being wound up with an inserted paper release liner. After air-cooling to ambient temperature a coated fabric having 0.75 mm by 0.6 mm (0.45 mm²) rectangular open apertures was obtained. Table 3 contains the process conditions and Table 4 contains the compositional information for Examples 1-2.

**Table 3. Examples 1-2 process conditions**

| Ex. | SIS Gel Input (barrel section number) | SALCARE Input (barrel section number) | Steel Roll Gap (mm) | Coating Speed (m/min) | Coating Weight (g/m²) |
|---|---|---|---|---|---|
| 1 | 2 | 4 | 0.37 | 2.1 | 147 |
| 2 | 2 | 4 | 0.25 | 2.1 | 78 |

**Table 4. Examples 1-2 compositions**

| Ex. | SIS (wt-%) | IRGANOX 1010 (wt-%) | SALCARE Type | SALCARE (wt-%) | KAYDOL oil (wt-%) |
|---|---|---|---|---|---|
| 1 | 18.0 | 0.6 | SC95 | 40.0 | 41.4 |
| 2 | 18.0 | 0.6 | SC91 | 40.0 | 41.4 |

### Adhesion and Asorbency of Examples

The gel coated PET fabrics (Examples 1-2) and 1 mm thick slabs having the compositions of Example 2 were tested for 180° peel adhesion from stainless steel using the peel test method described. The 180° peel adhesion from stainless steel was 0.1 N/dm for the gel slab (Example 2) and 0.0 N/dm for the gel coated fabric samples (Examples 1 and 2). The extremely low 180° peel adhesion demonstrates the inability of the composition and articles of the invention to form a strong adhesive bond. Consequently, the composition and articles of the invention are considered nonadherent or non-adhesive.

Examples 1-2 were tested for their ability to absorb 0.8 wt-% NaCl (saline). Samples (2.54 cm by 2.54 cm) of Examples 1 and 2 were soaked in saline. Absorbency was measured by the Saline Absorbency test as a function of time with the results in Table 6.

**Table 6. Saline absorbency vs. time for Examples 1-2**

| Ex. | SIS (wt-%) | SALCARE Type | 0.5 hour Saline Absorb. | 1 hour Saline Absorb. | 2 hours Saline Absorb. |
|---|---|---|---|---|---|
| 1 | 18.0 | SC95 | 1.9 | 2.2 | 2.6 |
| 2 | 18.0 | SC91 | 3.6 | 4.2 | 4.8 |

The saline absorbency data demonstrates that the composition and article of the invention can absorb an amount of saline that is 1-5 times their dry weight. All samples remained intact after saline exposure.

## Claims

1. A wound dressing comprising an apertured liquid permeable substrate and an absorbent, nonadherent polymer composition comprising:
a hydrophobic organic polymer matrix;
a plasticizing agent; and
hydrophilic organic microparticles.

2. The wound dressing of claim 1 wherein the hydrophobic organic polymer matrix comprises a styrene-isoprene-styrene copolymer, a styrene-butadiene-styrene copolymer, or mixtures thereof.

3. The wound dressing of claim 1 wherein the microparticles when in a substantially nonhydrated form have an average particle size of 10 µm or less.

4. The wound dressing of claim 1 wherein the apertured liquid permeable substrate comprises 1 to 225 apertures per square centimeter.

5. The wound dressing of claim 1 wherein the apertured liquid permeable substrate comprises apertures having an average opening size of 0.1 millimeter to 0.5 centimeter.

6. The wound dressing of claim 1 wherein the microparticles comprise an amine-containing organic polymer.

7. The wound dressing of claim 1 wherein the microparticles comprise a copolymer of sodium acrylate and acrylic acid.

8. The wound dressing of claim 1 wherein the polymer composition further comprises a bioactive agent.

9. The wound dressing of claim 1 wherein the polymer composition further comprises an additive selected from the group consisting of a tackifier, a crosslinking agent, a stabilizer, a compatibilizer, an extruding aid, a filler, a pigment, a dye, a swelling agent, a chain transfer agent, and combinations thereof.

10. The wound dressing of claim 1 wherein the hydrophobic organic polymer matrix comprises a mixture of two or more polymers.

11. The wound dressing of claim 1 wherein the microparticles are present in an amount of 1 wt-% to 60 wt-%, based on the total weight of the polymer composition.

12. The wound dressing of claim 1 wherein the hydrophobic organic polymer matrix comprises polyisobutylene, polyethylene-propylene rubber, polyethylene-propylene diene-modified rubber, polyisoprene, styrene-isoprene-styrene, styrene-butadiene-styrene, styrene-ethylene-propylene-styrnee, styrene-ethylene-butylene-styrene, and combinations thereof.

13. A wound dressing as defined in any of claims 1 to 12 for treating a wound.

14. Use of an absorbent, nonadherent polymer composition as defined in claims 1 for the preparation of a wound dressing comprising an apertured liquid permeable substrate and the absorbent, nonadherent polymer composition, for treating a wound.

## Patentansprüche

1. Wundverband umfassend ein mit Öffnungen versehenes flüssigkeitsdurchlässiges Substrat und eine absorbierende, nichthaftende Polymerzusammensetzung, umfassend:
eine hydrophobe organische Polymermatrix;
einen Weichmacher; und
hydrophile organische Mikroteilchen.

2. Wundverband nach Anspruch 1, wobei die hydrophobe organische Polymermatrix ein Styrol-Isopren-Styrol-Copolymer, ein Styrol-Butadien-StyrolCopolymer oder Mischungen davon umfaßt.

3. Wundverband nach Anspruch 1, wobei die Mikroteilchen eine durchschnittliche Teilchengröße von 10 µm oder weniger aufweisen, wenn sie in einer im Wesentlichen unhydratisierten Form vorliegen.

4. Wundverband nach Anspruch 1, wobei das mit Öffnungen versehene flüssigkeitsdurchlässige Substrat 1 bis 225 Öffnungen pro Quadratzentimeter umfaßt.

5. Wundverband nach Anspruch 1, wobei das mit Öffnungen versehene flüssigkeitsdurchlässige Substrat Öffnungen mit einer durchschnittlichen Öffnungsgröße von 0,1 Millimeter bis 0,5 Zentimeter umfaßt.

6. Wundverband nach Anspruch 1, wobei die Mikroteilchen ein aminhaltiges organisches Polymer umfassen.

7. Wundverband nach Anspruch 1, wobei die Mikroteilchen ein Copolymer von Natriumacrylat und Acrylsäure umfassen.

8. Wundverband nach Anspruch 1, wobei die Polymerzusammensetzung ferner ein bioaktives Mittel enthält.

9. Wundverband nach Anspruch 1, wobei die Polymerzusammensetzung ferner ein Additiv umfasst, das aus der Gruppe bestehend aus einem Klebrigmacher, einem Vernetzungsmittel, einem Stabilisator, einem Verträglichkeitsvermittler, einer Extrudierhilfe, einem Füllstoff, einem Pigment, einem Farbstoff, einem Quellmittel, einem Kettenübertragungsmittel und Kombinationen davon ausgewählt ist.

10. Wundverband nach Anspruch 1, wobei die hydrophobe organische Polymermatrix eine Mischung von zwei oder mehr Polymeren umfaßt.

11. Wundverband nach Anspruch 1, wobei die Mikroteilchen in einer Menge von 1 Gew.-% bis 60 Gew.-%, bezogen auf das Gesamtgewicht der Polymerzusammensetzung, vorliegen.

12. Wundverband nach Anspruch 1, wobei die hydrophobe organische Polymermatrix Polyisobutylen, Polyethylen-propylen-Kautschuk, dienmodifizierten Polyethylen-propylen-Kautschuk, Polyisopren, Styrol-Isopren-Styrol, Styrol-Butadien-Styrol, Styrol-Ethylen-Propylen-Styrol, Styrol-Ethylen-Butylen-Styrol und Kombinationen davon umfaßt.

13. Wundverband nach einem der Ansprüche 1 bis 12 zur Behandlung einer Wunde.

14. Verwendung einer absorbierenden, nichthaftenden Polymerzusammensetzung wie in Anspruch 1 definiert zur Herstellung eines Wundverbands, der ein mit Öffnungen versehenes flüssigkeitsdurchlässiges Substrat und die absorbierende, nichthaftende Polymerzusammensetzung umfasst, zur Behandlung einer Wunde.

## Revendications

1. Pansement comprenant un substrat perméable aux liquides pourvu d'ouvertures et une composition polymère absorbante, non adhérente, comprenant :
une matrice polymère organique hydrophobe ;
un agent plastifiant ; et
des microparticules organiques hydrophiles.

2. Pansement selon la revendication 1, dans lequel la matrice polymère organique hydrophobe comprend un copolymère de styrène-isoprène-styrène, un copolymère de styrène-butadiène-styrène, ou des mélanges de ceux-ci.

3. Pansement selon la revendication 1, dans lequel les microparticules, quand elles sont sous une forme fondamentalement non hydratée, ont une taille moyenne de particule de 10 µm ou moins.

4. Pansement selon la revendication 1, dans lequel le substrat perméable aux liquides pourvu d'ouvertures comprend de 1 à 225 ouvertures par centimètre carré.

5. Pansement selon la revendication 1, dans lequel le substrat perméable aux liquides pourvu d'ouvertures comprend des ouvertures ayant une taille moyenne d'ouverture de 0,1 millimètre à 0,5 centimètre.

6. Pansement selon la revendication 1, dans lequel les microparticules comprennent un polymère organique contenant une amine.

7. Pansement selon la revendication 1, dans lequel les microparticules comprennent un copolymère d'acrylate de sodium et d'acide acrylique.

8. Pansement selon la revendication 1, dans lequel la composition polymère comprend en outre un agent bioactif.

9. Pansement selon la revendication 1, dans lequel la composition polymère comprend en outre un additif sélectionné dans le groupe constitué d'un agent collant, d'un agent de réticulation, d'un stabilisant, d'un agent de compatibilisation, d'une aide d'extrusion, d'une charge, d'un pigment, d'un colorant, d'un agent gonflant, d'un agent de transfert de chaîne, et de combinaisons de ceux-ci.

10. Pansement selon la revendication 1, dans lequel la matrice polymère organique hydrophobe comprend un mélange de deux polymères ou plus.

11. Pansement selon la revendication 1, dans lequel les microparticules sont présentes dans une quantité de 1 % en poids à 60 % en poids, ce pourcentage étant exprimé relativement au poids total de la composition polymère.

12. Pansement selon la revendication 1, dans lequel la matrice polymère organique hydrophobe comprend le polyisobutylène, le caoutchouc de polyéthylène-propylène, le caoutchouc de polyéthylène-propylène modifié par un diène, le polyisoprène, le styrène-isoprène-styrène, le styrène-butadiène-styrène, le styrène-éthylène-propylène-styrène, le styrène-éthylène-butylène-styrène, et des combinaisons de ceux-ci.

13. Pansement comme il est défini dans l'une quelconque des revendications 1 à 12, destiné à soigner une plaie.

14. Utilisation d'une composition polymère absorbante, non adhérente comme il est défini dans la revendication 1 pour la préparation d'un pansement comprenant un substrat perméable aux liquides pourvu d'ouvertures et la composition polymère absorbante, non adhérente, pour soigner une plaie.
